# EUROPÄISCHE PATENTSCHRIFT

(11) **EP 1 351 931 B1**
(45) Veröffentlichungstag und Bekanntmachung des Hinweises auf die Patenterteilung: **11.08.2004**
(21) Anmeldenummer: 02702241.7
(22) Anmeldetag: 08.01.2002
(51) Int. Cl.: C07D 201/18, C07D 223/10, C07D 227/087

(54) **VERFAHREN ZUR LAGERUNG UND ZUM TRANSPORT VON N-VINYL-$g(e)- CAPROLACTAM**
METHOD FOR STORING AND TRANSPORTING N-VINYL-$g(e)-CAPROLACTAM
PROCEDE POUR STOCKER ET POUR TRANSPORTER DU N-VINYL-$g(e)-CAPROLACTAME

(30) Priorität: 10.01.2001 DE 10100752
(43) Veröffentlichungstag der Anmeldung: 15.10.2003
(73) Patentinhaber: BASF AKTIENGESELLSCHAFT, 67056 Ludwigshafen (DE)
(72) Erfinder: BOTTCHER, Arnd, 67227 Frankenthal (DE); PINKOS, Rolf, 67089 Bad Dürkheim (DE); LORENZ, Rudolf, Erich, 67063 Ludwigshafen (DE)
(86) Internationale Anmeldenummer: PCT/EP2002/000086
(87) Internationale Veröffentlichungsnummer: WO 2002/055493

(56) Entgegenhaltungen:
- WO-A-96/11217
- DE-A- 10 121 976
- DE-A- 19 906 316
- DE-B- 1 111 193
- US-A- 3 377 340
- US-A- 4 649 174
- US-A- 6 147 145

## Beschreibung

Die vorliegende Erfindung betrifft ein Verfahren zur Lagerung und zum Transport von N-Vinyl-ε-caprolactam.

N-Vinyl-ε-caprolactam findet unter anderem Verwendung als Monomerbaustein in Oligomeren, Polymeren und Copolymeren. Es findet somit beispielsweise Eingang in der Herstellung von Papierbeschichtungen, Klebstoffen, Druckfarben, Waschmitteln, Motorölzusätzen, Textilhilfsmitteln, strahlungshärtenden Lacken, Kosmetika, Pharmazeutika, Hilfsmitteln für die Erdölförderung oder Chemikalien für fotographische Anwendungen.

Die technische Herstellung von N-Vinyl-E-caprolactam erfolgt im Allgemeinen durch Addition von Ethin an Caprolactam und ist beispielsweise in Ullmanns Encyclopädie der technischen Chemie, Hrsg. Bartholomé et al., Band 23, 4. Auflage, Weinheim 1983, Seiten 611 bis 614 beschrieben. An die Synthese schließt sich üblicherweise eine destillative Reinigung an, bei der das Produkt durch Kondensation aus der Gasphase in hoher Reinheit gewonnen werden kann. Um unerwünschte Reaktionen wie etwa Zersetzung, Oligomerisierung oder Polymerisierung bei der Lagerung und beim Transport zu unterbinden wird üblicherweise ein Stabilisator zugegeben. Ein häufig eingesetzter Stabilisator ist N,N'-Bis(1-methylpropyl)-1,4-phenylendiamin, welcher von der BASF AG unter dem Handelsnamen Kerobit® BPD vertrieben wird. Weitere bekannte Stabilisatoren sind beispielsweise Alkalihydroxide oder Phenothiazin-Derivate.

Bei Anwendungen, bei denen die Eigenfärbung des Produkts möglichst gering sein sollte, wie beispielsweise im kosmetischen oder fotographischen Bereich oder bei Papierbeschichtungen, ist neben einer hohen chemischen Reinheit auch eine sehr hohe Reinheit in Bezug auf farbgebende Verunreinigungen gefragt. Bei farbgebenden Verunreingungen reichen im Allgemeinen Mengen von wenigen Gew.-ppb aus, um deutliche Verfärbungen des Produkts zu erreichen.

Das nach der Destillation erhaltene und in der Regel mit einem Stabilisator versetzte N-Vinyl-E-caprolactam wird üblicherweise in flüssigem Zustand in Behälter wie etwa Flaschen, Fässer oder Container gefüllt und bei Umgebungstemperatur gelagert und transportiert. Aufgrund seines Schmelzpunktes von 35°C erstarrt das N-Vinyl-ε-caprolactam kurze Zeit nach der Befüllung. Vor der Entleerung der Behälter wird die feste Masse üblicherweise wieder aufgeschmolzen. Dabei wurde erfindungsgemäß erkannt, daß das aufgeschmolzene Produkt eine deutlich dunklere, gelbliche bis bräunliche Farbe aufweist als das ursprüngliche Einsatzprodukt. Diese unerwünschte Farbänderung wird im folgenden als Verfärbung bezeichnet.

Es bestand daher die Aufgabe, ein Verfahren zur Lagerung und zum Transport von N-Vinyl-E-caprolactam zu finden, welches die oben beschriebenen Nachteile nicht mehr besitzt und auch nach längerer, mehrmonatiger Lager- und Transportzeit nur eine sehr geringe Tendenz zur Verfärbung zeigt.

DE 1 111 193 beschreibt die Lagerung von N-unsubstituierten Lactamen bei erhöhter Temperatur.

US 6 147 145 und DE 199 06 316 beschreiben die Lagerung von stabilisierten N-Vinylverbindungen.

Es wurde überraschenderweise ein Verfahren zur Lagerung und zum Transport von N-Vinyl-ε-caprolactam gefunden, das dadurch gekennzeichnet ist, daß man das N-Vinyl-ε-caprolactam in flüssiger Phase hält.

Die flüssige Phase wird im Allgemeinen auch als "Schmelze" bezeichnet. Sie kann beim erfindungsgemäßen Verfahren auch mit Feststoffpartikeln wie etwa kristallinem N-Vinyl-E-caprolactam durchsetzt sein. Des weiteren können Feststoffpartikel wie etwa kristallines N-Vinyl-E-caprolactam beispielsweise als Bodensatz vorliegen. Beim erfindungsgemäßen Verfahren hält man bevorzugt den überwiegenden Teil des zu lagernden beziehungsweise zu transportierenden N-vinyl-ε-caprolactams in der flüssigen Phase. Besonders bevorzugt hält man über 90 Gew.-%, ganz besonders bevorzugt über 99 Gew.-% und insbesondere das gesamte N-vinyl-ε-caprolactam in der flüssigen Phase. Je höher der Anteil der flüssigen Phase gegenüber einer eventuell vorliegenden festen Phase ist, desto geringer ist in der Regel die Tendenz zur Verfärbung.

Um das Produkt in flüssiger Phase zu halten, wendet man im Allgemeinen eine Temperatur an, die dem Schmelzpunkt entspricht oder oberhalb des Schmelzpunktes liegt. Reines N-Vinyl-E-caprolactam besitzt einen Schmelzpunkt von 35°C. Beim erfindungsgemäßen Verfahren hält man das N-Vinyl-ε-caprolactam bevorzugt bei einer Temperatur von 35 bis 100°C, besonders bevorzugt bei 35 bis 75°C und ganz besonders bevorzugt bei 35 bis 60°C.

Die Zeitdauer für die Lagerung und den Transport beträgt beim erfindungsgemäßen Verfahren im Allgemeinen wenige Stunden bis mehrere Monate. Sie kann gegebenenfalls auch kürzer oder länger sein. Bevorzugt beträgt die Zeitdauer mindestens drei Tage, besonders bevorzugt mindestens fünf Tage und ganz besonders bevorzugt mindestens sieben Tage.

Das beim erfindungsgemäßen Verfahren eingesetzte N-Vinyl-E-caprolactam kann mit Stabilisatoren zur Unterbindung unerwünschter Reaktionen wie etwa Zersetzung, Oligomerisierung oder Polymerisierung versetzt sein. Als Beispiele geeigneter Stabilisatoren seien N,N'-Bis(1-methylpropyl)-1,4-phenylendiamin, welcher von der BASF AG unter dem Handelsnamen Kerobit® BPD vertrieben wird, Alkalihydroxide oder Phenothiazin-Derivate genannt. Ist das N-Vinyl-ε-caprolactam mit Stabilisatoren versetzt, so liegt deren Gehalt im Allgemeinen bei 1 bis 1000 Gew.-ppm, bevorzugt bei 1 bis 100 Gew.-ppm und besonders bevorzugt bei 5 bis 50 Gew.-ppm.

Das beim erfindungsgemäßen Verfahren eingesetzte N-Vinyl-E-caprolactam besitzt im Allgemeinen eine Reinheit von über 98 Gew.-%, bevorzugt von über 99 Gew.-% und besonders bevorzugt von über 99,5 Gew.-%. Bei dem auf 100 Gew.-% fehlenden Anteil handelt es sich im Allgemeinen um nicht umgesetztes Caprolactam, um zugesetzte Stabilisatoren oder um Verunreinigungen wie sie beispielsweise bei der Herstellung des Produkts oder beispielsweise durch Zersetzung, Oligomerisierung oder Polymerisierung entstehen. In der Regel handelt es sich bei dem fehlenden Anteil überwiegend um Caprolactam.

Das N-Vinyl-ε-caprolactam kann beim erfindungsgemäßen Verfahren mit verschiedenen Gasen überschichtet sein beziehungsweise verschiedene Gase enthalten. Als Beispiele seien Stickstoff, Edelgase (z.B. Argon) oder Luft genannt.

Für die Lagerung und den Transport setzt man im Allgemeinen Behälter ein. Ihre Größe ist für das erfindungsgemäße Verfahren im Allgemeinen unerheblich. In der Regel setzt man Behälter im L-und m³-Bereich ein. Auch die geometrische Form der Behälter ist für das erfindungsgemäße Verfahren im Allgemeinen unerheblich. Bevorzugte Beispiele sind: (i) im Wesentlichen kugelförmige Behälter (z.B. sogenannte Kugeltanks), (ii) im Wesentlichen zylinderförmige Behälter (z.B. Flaschen, Fässer, sogenannte Zylindertanks oder Kesselwagen) und (iii) im Wesentlichen würfel- oder quaderförmige Behälter.

Die Wandung des Behälters sollte gegenüber N-Vinyl-ε-caprolactam chemisch inert und dicht sein. Geeignete Materialen sind beispielweise Eisen, Edelstahl oder innenlackierte Metall- oder Kunststoffgebinde (z.B. aus Polypropylen oder Polyethylen).

Bevorzugt lagert und transportiert man beim erfindungsgemäßen Verfahren das N-Vinyl-E-caprolactam in einem wärmeisolierten Behälter. Darunter sind Behälter zu verstehen, welche eine wärmeisolierende Dämmung umfassen. Als geeignete wärmeisolierende Dämmungen sind beispielsweise Zwischenschichten aus Vakuum, aus einem gering wärmeleitfähigen Gas wie etwa Helium, oder aus festen Dämmmaterialien wie etwa geschäumtes Polystyrol (z.B. Styropor® ), Glas- oder Mineralwolle genannt.

Bei der Lagerung und dem Transport von N-Vinyl-E-caprolactam in einem wärmeisolierten Behälter kann sich das N-Vinyl-E-caprolactam (i) direkt ohne weitere Behälter oder (ii) verpackt in weiteren Behältern befinden. Als geeignete Beispiele zu (i) seien wärmeisolierte Tanks (z.B. Kugel-, Zylinder- oder Kesseltanks) oder wärmeisolierte Fässer genannt. Als geeignete Beispiele zu (ii) seien wärmeisolierte Container (z.B. Iso-Container), welche das N-Vinyl-E-caprolactam in Form von weiteren Behältern (z.B. Fässer oder Flaschen) enthalten, genannt.

Besonders bevorzugt lagert und transportiert man beim-erfindungsgemäßen Verfahren das N-Vinyl-ε-caprolactam in einem wärmeisolierten und beheizbaren Behälter. Darunter sind Behälter zu verstehen, welche neben der oben genannten Wärmeisolierung eine Heizung enthalten. Bei wärmeisolierten Behältern, in denen sich das N-Vinyl-E-caprolactam (i) direkt ohne weitere Behälter befindet, kann sich die Heizung beispielsweise in Form von Heizelementen direkt im zu lagernden oder zu transportierenden N-Vinyl-E-caprolactam befinden. Eine andere Möglichkeit besteht beispielsweise darin, daß sich die Heizung zwischen der Wärmeisolierung und der innenliegenden Behälterschicht befindet. Als Beispiele hierzu seien mit einem Heizmantel versehene Fässer oder Tanks genannt. Bei wärmeisolierten Behältern, in denen sich das N-Vinyl-E-caprolactam (ii) verpackt in weiteren Behältern befindet, befindet sich die Heizung üblicherweise im Inneren dieser Behälter.

In einer bevorzugten Ausführungsform des erfindungsgemäßen Verfahrens werden 220 L-Fässer bei 40°C in einem beheizbaren Iso-Container gelagert beziehungsweise transportiert.

Das erfindungsgemäße Verfahren zur Lagerung und zum Transport von N-Vinyl-E-caprolactam ist besonders überraschend, da nach allgemeinem Fachwissen gerade in der flüssigen und somit diffusionsmobilen Phase eine wesentlich größere Reaktivität zu erwarten ist.

Das bevorzugte erfindungsgemäße Verfahren, bei dem man das N-Vinyl-E-caprolactam bei der Schmelztemperatur des N-Vinyl-E-caprolactams oder darüber hält, ist insbesondere überraschend, da nach allgemeinem Fachwissen die Reaktivität in der Regel mit der Temperatur steigt.

Sowohl die Tatsache, daß sich das flüssige Produkt als weniger reaktiv in Bezug auf die Bildung farbgebender Verunreinigungen zeigt als das feste Produkt, als auch die Tatsache, daß dieser Effekt auch für flüssiges Produkt, welches bei deutlich höherer Temperatur gehalten wird als das feste Produkt, besteht, kann aus dem allgemeinen Fachwissen nicht abgeleitet werden.

Das erfindungsgemäße Verfahren ermöglicht die Lagerung und den Transport von N-Vinyl-ε-caprolactam, wobei das Verfahren auch nach längerer, mehrmonatiger Lager- und Transportzeit nur zu einer geringen Verfärbung des Produkts führt.

### Beispiele

Zur Charakterisierung der möglichen Verfärbung des N-Vinyl-E-caprolactams wurden die Farbzahlen nach APHA, analog DIN EN 1557 bestimmt.

### Beispiele 1 bis 2

Jeweils 500 g flüssiges N-Vinyl-ε-caprolactam mit einer Farbzahl nach APHA von 8 wurden in eine Glasflasche gegeben und verschlossen.

In Vergleichsbeispiel 1 wurde die Probe bei einer Umgebungstemperatur von ca. 25°C nach kurzer Zeit fest und 21 Tage bei dieser Temperatur im festen Zustand gelagert. Die Probe wurde anschließend bei etwa 50°C aufgeschmolzen und erneut die Farbzahl nach APHA bestimmt. Sie betrug 125.

In Beispiel 2 wurde die Probe im flüssigen Zustand bei 50°C gelagert und anschließend erneut die Farbzahl nach APHA bestimmt. Sie betrug 24.

### Beispiele 3 bis 4

Jeweils 200 kg flüssiges N-Vinyl-E-caprolactam mit einer Reinheit von 99,8 GC-Flächen-% und einer Farbzahl nach APHA von 58 wurden in ein 220 L-Eisenfaß gegeben und verschlossen.

In Vergleichsbeispiel 3 wurde die Probe bei einer Umgebungstemperatur von 20°C nach kurzer Zeit fest und 1 Monat bei dieser Temperatur im festen Zustand gelagert. Die Probe wurde anschließend bei etwa 50°C aufgeschmolzen und erneut die Farbzahl nach APHA bestimmt. Sie betrug 152.

In Beispiel 4 wurde die Probe im flüssigen Zustand bei 40°C gelagert und anschließend erneut die Farbzahl nach APHA bestimmt. Sie betrug 84.

Die Beispiele 1 bis 4 zeigen, daß das in flüssiger Phase gelagerte N-Vinyl-ε-caprolactam gegenüber dem in fester Phase gelagertem nur eine äußerst geringe Tendenz zur Verfärbung zeigt. So erhöhte sich etwa die Farbzahl beim erfindungsgemäßen Beispiel 4, bei dem das N-Vinyl-ε-caprolactam über einem Monat flüssig in einem 220 L-Eisenfaß gelagert wurde nur um 26 Einheiten nach APHA, wohingegen beim Vergleichsbeispiel 3 bei Lagerung in fester Phase eine deutlich größere Zunahme um 94 Einheiten beobachtet wurde.

## Patentansprüche

1. Verfahren zur Lagerung und zum Transport von N-Vinyl-E-caprolactam, **dadurch gekennzeichnet, daß** man einen wärmeisolierten und/oder beheizbaren Behälter einsetzt und das N-Vinyl-E-caprolactam bei einer Temperatur von 35 bis 100°C in flüssiger Phase hält.

2. Verfahren nach Anspruch 1, **dadurch gekennzeichnet, daß** man das N-Vinyl-ε-calprolactam bei einer Temperatur von 35 bis 60°C hält.

3. Verfahren nach den Ansprüchen 1 bis 2, **dadurch gekennzeichnet, daß** man das N-Vinyl-ε-caprolactam über eine Zeitdauer von mindestens fünf Tage lagert oder transportiert.

4. Verfahren nach den Ansprüchen 1 bis 3, **dadurch gekennzeichnet, daß** man das N-Vinyl-ε-caprolactam über eine Zeitdauer von mindestens sieben Tage lagert oder transportiert.

5. Verfahren nach den Ansprüchen 1 bis 4, **dadurch gekennzeichnet, daß** man N-Vinyl-ε-caprolactam mit einer Reinheit von über 98 Gew.-% einsetzt.

## Claims

1. A method of storing and transporting N-vinyl-ε-caprolactam, wherein a thermally insulated and/or heatable container is used and the N-vinyl-ε-caprolactam is kept in the liquid phase at a temperature of 35 to 100°C.

2. A method as claimed in claim 1 wherein the N-vinyl-ε-caprolactam is kept at a temperature of 35 to 60°C.

3. A method as claimed in claims 1 to 2 wherein the N-vinyl-ε-caprolactam is stored or transported for a period of at least five days.

4. A method as claimed in claims 1 to 3 wherein the N-vinyl-ε-caprolactam is stored or transported for a period of at least seven days.

5. A method as claimed in claims 1 to 4 wherein N-vinyl-ε-caprolactam with a purity of over 98% by weight is used.

## Revendications

1. Procédé pour stocker et transporter la N-vinyl-ε-caprolactame, **caractérisé en ce que** l'on met en oeuvre un récipient calorifugé et/ou calorifique et que l'on maintient la N-vinyl-ε-caprolactame en phase liquide à une température allant de 35°C à 100°C.

2. Procédé selon la revendication 1, **caractérisé en ce que** l'on maintient la N-vinyl-ε-caprolactame à une température allant de 35°C à 60°C.

3. Procédé selon les revendications 1 à 2, **caractérisé en ce que** l'on stocke ou que l'on transporte la N-vinyl-ε-caprolactame pendant une durée d'au moins cinq jours.

4. Procédé selon les revendications 1 à 3, **caractérisé en ce que** l'on stocke ou que l'on transporte la N-vinyl-ε-caprolactame pendant une durée d'au moins sept jours.

5. Procédé selon les revendications 1 à 4, **caractérisé en ce que** l'on met en oeuvre la N-vinyl-ε-caprolactame ayant une pureté de plus de 98% en poids.
